# EUROPÄISCHE PATENTANMELDUNG

(11) **EP 2 213 719 A1**
(43) Veröffentlichungstag der Anmeldung: **04.08.2010**
(21) Anmeldenummer: 10000684.0
(22) Anmeldetag: 25.01.2010
(51) Int. Cl.: C12M 1/00

(54) **Anordnung und Verfahren zur Biomasseerzeugung**

(30) Priorität: 28.01.2009 DE 102009006490
(71) Anmelder: Universität Duisburg-Essen, 45141 Essen (DE)
(72) Erfinder: Franke, Hilmar, 49566 Bramsche (DE); Kleinefeld, Theo, 46240 Bottrop (DE); Junge, Helmut, 47167 Duisburg (DE)
(74) Vertreter: Gesthuysen, von Rohr & Eggert

(57) **Zusammenfassung**

Es werden eine Anordnung und ein Verfahren zur Biomasseerzeugung in einem flüssigen Medium mit einer lebenden Spezies, insbesondere Algen, vorgeschlagen. Die Bioznasseerzeugung wird dadurch optimiert, daß der pH-Wert des Mediums in einem Bereich von 6,5 bis 8,5 gehalten, zyklisch variiert und/oder durch Steuerung der Zugabe von Kohlendioxid gesteuert oder geregelt wird,

## Beschreibung

Die vorliegende Erfindung betrifft eine Anordnung und ein Verfahren zur Biomasseerzeugung.

Die vorliegende Erfindung befaßt sich insbesondere mit der Nachahmung der Photosynthese zur Bindung von Kohlendioxid. Es ist bekannt, hierzu eine Algensuspension zu beleuchten, um das Wachstum der Algen anzuregen. Die Algen verbrauchen Kohlendioxid zum Wachstum und zur Bildung von Biomasse.

Der vorliegenden Erfindung liegt die Aufgabe zugrunde, eine Anordnung und ein Verfahren zur Biomasseerzeugung anzugeben, wobei auf einfache Weise optimiert Wachstumsbedingungen erreicht oder eingehalten werden können bzw. die Biomasseerzeugung optimiert werden kann.

Die obige Aufgabe wird durch eine Anordnung gemäß Anspruch 1 oder ein Verfahren gemäß Anspruch 6 gelöst. Vorteilhafte Weiterbildungen sind Gegenstand der Unteransprüche.

Ein Aspekt der vorliegenden Erfindung liegt darin, den pH-Wert eines Mediums mit einer lebenden Spezies, wie Algen, durch die Zuführung von Kohlendioxid und/oder anderen Kohlenwasserstoffen zu steuern oder zu regeln, So können optimale Wachstumsbedingungen für die lebende Spezies auf überraschend einfache Art und Weise erreicht werden.

Gemäß einem anderen Aspekt der vorliegenden Erfindung wird der pH-Wert des Mediums mit mindestens einer lebenden Spezies zyklisch variiert. Dies ist einem optimalen Wachstum der Spezies bzw. einer optimierten Biomasseerzeugung zuträglich.

Gemäß einem weiteren Aspekt der vorliegenden Erfindung wird der pH-Wert des vorzugsweise Wasser enthaltenden oder aus Wasser bestehenden Mediums mit der lebenden Spezies zumindest im wesentlichen zwischen 6,5 und 8,5, insbesondere zwischen 7 und 8, gehalten. Dies ist einer optimalen Aufnahme von Kohlendioxid einerseits und guten Wachstumsbedingungen für die lebende Spezies, insbesondere von Algen, andererseits zuträglich. So kann eine optimierte Biomasseerzeugung erreicht werden.

Die vorgenannten Aspekte sowie die weiteren Aspekte und Merkmale der vorliegenden Erfindung, die nachfolgend beschrieben werden, können unabhängig voneinander und in beliebiger Kombination realisiert werden.

Weitere Aspekte, Vorteile, Merkmale und Eigenschaften der vorliegenden Erfindung ergeben sich aus den Ansprüchen und der nachfolgenden Beschreibung bevorzugter AusEiihrungsformen anhand der Zeichnung. Es zeigt:
- Fig. 1: eine schematische Darstellung einer vorschlagsgemäßen Anordnung gemäß einer ersten Ausführungsform;
- Fig. 2: eine schematische Darstellung einer vorschlagsgemäßen Anordnung gemäß einer zweiten Ausfühmngsform,;
- Fig. 3: einen schematischen, nicht maßstabsgerechten Schnitt einer lichtleitenden Faser der Anordnung gemäß Fig. 2;
- Fig. 4: einen schematischen, nicht maßstabsgerechten Schnitt einer anderen lichtleitenden Faser der Anordnung gemäß Fig. 2; und
- Fig. 5: eine schematische Darstellung einer vorschlagsgemäßen Anordnung gemäß einer dritten Ausführungsform.

In den Figuren werden für gleiche oder ähnliche Komponenten, Bauteile und dgl. die gleichen Bezugszeichen verwenden. Insbesondere ergeben sich entsprechende oder vergleichbare Vorteile, auch wenn eine wiederholte Beschreibung weggelassen ist.

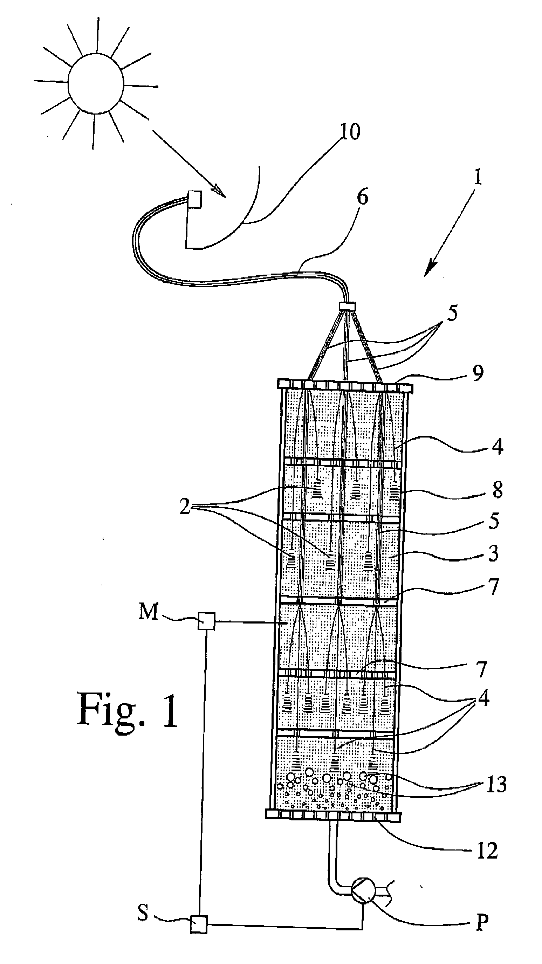

Fig. 1 zeigt in einer schematischen, nicht maßstabsgerechten Darstellung eine vorschlagsgemäße Anordnung 1 gemäß einer ersten Ausführungsform. Die Anordnung 1 dient der Biomasseerzeugung, wobei Licht 2 (schematisch in Fig. 1 angedeutet) und/oder Kohlendioxid bzw. sonstige Kohlenwasserstoffe einem flüssigen Medium 3 zugeführt werden, das mindestens eine lebende Spezies enthält.

Dem Medium 3 wird besonders bevorzugt Kohlendioxid, insbesondere als Gas, ggf auch als Komponente eines Gasgemisches, und/oder auf sonstige Weise, beispielsweise in flüssiger bzw, gelöster Form zugeführt. Alternativ oder zusätzlich können auch sonstige Kohlenwasserstoffe dem Medium 3 zugeführt werden. Dies kann in entsprechender Weise oder anstatt der Zufiihrung von Kohlendioxid erfolgen. Dementsprechend gelten die Ausführungen zu der Zuführung von Kohlendioxid auch in entsprechender Weise für die Zuführung von sonstigen Kohlenwasserstoffen. In der nachfolgenden Beschreibung wird der Einfachheit halber jedoch oft nur von der Zuführung von Kohlendioxid gesprochen.

Bei dem Medium 3 handelt es sich insbesondere um eine Suspension oder dergleichen. Dementsprechend ist der Begriff "flüssig" in einem weiten Sinne zu verstehen, so daß insbesondere Suspensionen, Dispersionen oder sonstige Gemische oder Stoffe mit flüssigen Phasen oder Anteilen umfaßt sind.

Das Medium 3 ist vorzugsweise photoaktiv und/oder biologisch aktiv. Insbesondere kann in dem Medium 3 eine Photosynthese oder eine sonstige Licht 2 und/oder Kohlendioxid benötigende Reaktion ablaufen. Insbesondere enthält das Medium 3 hierzu eine biologisch aktive Spezies, insbesondere Algen, sonstige Bakterien oder dergleichen.

Insbesondere ist das Medium 3 wäßrig bzw, enthält Wasser und/oder stark lichtbestreuend.

Besonders bevorzugt wird eine Algensuspension als Medium 3 eingesetzt. Insbesondere enthält das Medium 3 eine Algenmischkultur, die vorzugsweise zumindest in ähnlicher Form in Flüssen, Teichen oder dergleichen vorkommt. Derartige Mischkulturen sind nämlich besonders resistent gegen Umwelteinflüsse, Krankheiten und/oder sonstige Störungen.

Da die Anordnung 1 vorzugsweise mit Algen bzw. mit einer Algensuspension als Medium 3 eingesetzt wird, wird der folgenden Beschreibung primär auf das durch das eingeleitete Licht 2 induzierte Algenwachstum abgestellt. Jedoch gelten diese Ausführungen entsprechend auch für sonstige Spezies oder photo- bzw. bioaktive Medien 3.

Die Anordnung 1 weist eine Beleuchtungseinrichtung, hier mit lichtleitenden Fasern 4, zur Zuleitung des Lichts 2 auf Bei der ersten Ausführungsform enden mehrere oder alle Fasern 4 in verschiedenen Raumbereichen, insbesondere zumindest teilweise in unterschiedlichen Tiefen und/oder Vertikalebenen, in dem Medium 3. Dies gestattet eine dreidimensionale Verteilung des Lichts 2 in dem Medium 3, wie in Fig. 1 beispielhaft schematisch angedeutet.

Vorzugsweise sind die Fasern 4 flexibel. Jedoch können grundsätzlich auch starre lichtleitende Stäbe - zumindest abschnittsweise und/oder am Austrittsende ins Medium 3 - eingesetzt werden.

Besonders bevorzugt enden die Fasern 4 in verschiedenen Ebenen innerhalb des Mediums 3. Jedoch können die Fasern 4 auch alle unterschiedlich oder unregelmäßig enden.

Ausgehend von einem Bündel (Hauptbündel) 5 von Fasern 4 erfolgt insbesondere eine Verzweigung oder Verästelung in Einzelbündel 6 und/oder Einzeifasern 4. Um eine optimale Verteilung des Lichts 2 in dem Medium 3 zu erreichen, enden die Fasern 4 vorzugsweise einzeln, also separat voneinander in dem Medium 3. Jedoch können auch mehrere Fasern 4 zusammen, beispielsweise als Einzelbündel 5, in dem Medium 3 enden.

Das Licht tritt zumindest im wesentlichen oder ausschließlich am Ende der jeweiligen Faser 4 aus und beleuchtet das Medium 3 im jeweiligen Raumbereich, wie durch Striche in Fig. 1 angedeutet.

Je nach Biegung bzw. Krümmung, Material, Oberfläche, Oberflächenaufrauhung oder dergleichen können die Fasern 4 auch mehr oder weniger Licht seitlich entlang der jeweiligen Faser 4 abgeben. Auch so kann eine optimierte dreidimensionale Verteilung von Licht 2 in dem Medium 3 erfolgen. Bei entsprechender Ausgestaltung der Fasern 4 ist es sogar möglich, daß zumindest im wesentlichen das Gesamtlicht bereits über die im Medium 3 verlaufenden Längen der jeweiligen Faser 4 seitlich abgegeben wird und gegebenenfalls nur noch ein verhältnismäßig geringer Anteil am Ende der jeweiligen Faser 4 in das Medium 3 abgestrahlt wird. In diesem Fall verlaufen die Fasern 4 vorzugsweise im wesentlichen über längere Strecken parallel zueinander oder beispielsweise gewendelt oder mäanderförmig durch das Medium 3 und können gegebenenfalls sogar alle in einer Ebene oder Tiefe in dem Medium 3 enden.

Versuche haben gezeigt, daß die Lichtaustrittsflächen - insbesondere also die Enden - der Fasern 4 von den Algen nicht besiedelt werden. Dementsprechend ist keine übermäßige Reinigung oder Befreiung von Algen erforderlich. Vielmehr ergibt sich ein selbstorganisierendes System, das möglichst effizient das eingeleitete Licht 2 zum Algenwachstum ausnutzt.

Die Fasern 4 können lose in das Medium 3 eintauchen bzw. in dieses hineinhängen, gegebenenfalls auch als Bündel 5 oder 6, die sich dann insbesondere verzweigen.

Vorzugsweise weist die Anordnung 1 insbesondere gitter- oder stangen artige Halterungen 7 in dem Medium 3 auf, die insbesondere in verschiedenen Ebenen oder Tiefen angeordnet sind, um die Fasern 4 bzw. Bündel 5, 6 zu halten oder zu führen.

Besonders bevorzugt bilden die Haltezungen 7 Zwischenböden in einem Behälter oder Tank 8 mit dem Medium 3, um die Fasern 4 oder Bündel 5 oder 6 in verschiedenen Raumbereiches innerhalb des Mediums 3 enden zu lassen.

Bedarfsweise können die Fasern 4 bzw. Bündel 5, 6 zumindest in Endbereichen im Medium 3, insbesondere durch eine Strömung des Mediums 3, beweglich sein.

Die Zuführung oder Einleitung der Fasern 4 bzw. Bündel 5/6 erfolgt vorzugsweise von oben, beim Ausführungsbeispiel durch eine obere Halterung oder Abdeckung 9 oder dergleichen.

Die Fasern 4 bzw. Bündel 5/6 erstrecken sich vorzugsweise zumindest im wesentlichen vertikal von oben nach unten im Medium 3. Dies ist insbesondere dann vorteilhaft, wenn die Dichte der Fasern 4 größer als die Dichte des Mediums 3 ist, diese also nicht aufschwimmen.

Jedoch ist es grundsätzlich auch möglich, daß sich die Fasern 4 bzw. Bündel 5/6 von unten nach oben in das Medium 3 und/oder auf sonstige Weise - beispielsweise quer - in das Medium 3 erstrecken oder zumindest abschnittsweise derart gerührt sind.

Die Fasern 4 bestehen vorzugsweise aus Kunststoff, Glas oder einem sonstigen zur Lichtleitung geeigneten Material oder Aufbau. Insbesondere können auch Verbundwerkstoffe oder Verbundaufbauten, beispielsweise mit Beschichtungen, verwendet werden.

Alternativ - also auch unabhängig - oder zusätzlich zu der dreidimensionalen Verteilung des Lichts 2 im Medium 3 durch die Fasern 4 gestatten die Fasern 4 bzw. Bündel 5/6 eine optimierte Zuführung des Lichts 2.

Beim Darstellungsbeispiel wird vorzugsweise Sonnenlicht zur Beleuchtung des Mediums 3 bzw. der Algen eingesetzt, auch wenn grundsätzlich jedes sonstige Licht verwendet werden kann. In diesem Zusammenhang ist anzumerken, daß der Begriff "Licht" vorzugsweise in einem weiten Sinne dahingehend zu verstehen ist, daß nicht nur sichtbares Licht, sondern beispielsweise alternativ oder zusätzlich auch Infrarot-Licht und/oder ultraviolettes Licht entsprechend dem Medium 3 zugeführt werden kann. Generell kann die Energiezuführung über die Fasern 4 des Medium 3 auch anderen Zwecken als der beim Darstellungsbexspiel vorgesehenen Photosynthese und/oder Anregung des biologischen Wachstums und/oder der Umwandlung von Kohlendioxid in Biomasse dienen.

Die Anordnung 1 bzw. Beleuchtungseinrichtung weist vorzugsweise eine Lichtsammeleinrichtung, insbesondere einen Lichttrichter oder Kollektorspiegel 10, zum Auffangen von Sonnenlicht auf, wie in Fig. 1 schematisch dargestellt. Das aufgefangene Sonnenlicht oder sonstiges Licht kann dann über die Fasern 4 bzw. insbesondere ein Bündel 5 oder 6 zu dem Behälter bzw. Tank 8 mit dem Medium 3 mit insbesondere sehr geringen Verlusten geleitet werden. Insbesondere sind so Zuleitungen von mehreren 100 m möglich. Dies ist insbesondere bei großem Lichtbedarf sehr vorteilhaft, da entsprechend große Flächen, Lichtsammeleinrichtungen oder dergleichen erforderlich sind und/oder entsprechende bauliche Gegebenheiten berücksichtigt werden müssen und/oder können. Insbesondere ist es so möglich, den Behälter bzw. Tank 8 mit dem Medium 3 in einem nicht dargestellten Gebäude oder dergleichen unterzubringen und beispielsweise trotzdem Sonnenlicht zur Beleuchtung einzusetzen.

Die Anordnung 1 weist vorzugsweise eine Versorgungseinrichtung 11 zur Zuführung von Kohlendioxid und/oder sonstigen Kohlenwasserstoffen, insbesondere zur Einleitung von Gas, das insbesondere Kohlendioxid enthält oder daraus besteht, auf. Beispielsweise kann Rauchgas, Klärgas, Faulgas, Luft oder dergleichen eingeleitet werden. Beim Darstellungsbeispiel weist die Einrichtung 11 insbesondere einen Boden, ein Sieb 12 oder ein sonstiges geeignetes Einleitungsrnittel auf, um das Gas insbesondere in Form von Gasblasen 13 in das Medium 3 einzuleiten bzw. abzugeben, wie in Fig. 1 schematisch angedeutet.

Die Gaszuleitung oder -einleitung erfolgt insbesondere bodenseitig und/oder in einer gewissen Tiefe, vorzugsweise von mindestens 5 bis 6 m, um einen guten Gasaustausch bzw. eine gute Versorgung des Mediums 3 und damit der Algen mit dem Gas zu ermöglichen.

Bedarfsweise wird das Gas oberhalb des Mediums 3 - beim Darstellungsbeispiel beispielsweise oberhalb der Abdeckung 9 oder innerhalb des Behälters bzw. Tanks 8 abgesaugt und wieder über die Einrichtung 11 zugeleitet, also rezirkuliert, um eine besonders gute Ausnutzung bzw. einen besonders guten Abbau des Gases, insbesondere des Kohlendioxids, zu erreichen.

Alternativ oder zusätzlich kann die Zuführung von Kohlendioxid bzw. sonstigen Kohlenwasserstoffen auch in flüssiger bzw. wäßriger oder gelöster Form erfolgen. Diese Art der Zuführung kann auch mit der gasförmigen Zuführung gekoppelt werden.

Die Anordnung 1 bzw. Biomasseerzeugung erfolgt vorzugsweise bei einem pH-Wert des Mediums 3 von insbesondere etwa 6,5, insbesondere 7 oder mehr, und/oder von höchstens 8,5, insbesondere 8 oder weniger. Besonders bevorzugt erfolgt eine Regelung des pH-Werts insbesondere derart, daß der pH-Wert zumindest im wesentlichen in den genannten Grenzen bzw. in dem genannten pH-Wert-Bereich gehalten wird und/oder zyklisch variiert wird.

Die Steuerung bzw. Regelung des pH-Werts des Mediums 3 erfolgt vorzugsweise durch entsprechende Steuerung der Versorgungsleitung 11 und/oder durch Variieren und/oder Unterbrechen der Zuführung von Kohlendioxid und/oder anderen Kohlenwasserstoffen. Besonders bevorzugt wird ein Regelkreis gebildet. Der pH-Wert des Mediums 3 wird besonders bevorzugt durch Steuerung der Zugabe von Kohlendioxid in insbesondere sehr kleinen Gasblasen 13 gesteuert bzw. geregelt. Bei Zugabe von Kohlendioxid wird dieses nämlich im Wasser gelöst, wodurch der pH-Wert abgesenkt wird.

Beim Darstellungsbeispiel weist die Anordnung 1 eine Steuereinrichtung S auf, die die Zuführung von Kohlendioxid und/oder anderen Kohlenwasserstoffen bzw. die Versorgungseinrichtung 11 und/oder eine Pumpe P o. dgl. steuert. Insbesondere kann die Steuereinrichtung S die Zuführung von Kohlendioxid bzw. anderen Kohlenwasserstoffen in gewünschter Weise variieren und/oder unterbrechen, um den pH-Wert des Mediums 3 zu steuern bzw. zu regeln.

Um die Regelgröße, also den pH-Wert des Mediums 3, zu erfassen, weist die Anordnung 1 bzw. die Steuereinrichtung S vorzugsweise eine Meßeinrichtung M auf, die beispielsweise über einen entsprechenden Sensor o. dgl. den pH-Wert des Mediums 3 oder einen dazu korrespondierenden bzw. damit korrelierten Wert mißt. Jedoch sind auch andere konstruktive Lösungen möglich.

Die Steuereinrichtung S steuert die Zuführung von Kohlendioxid bzw. sonstigen Kohlenwasserstoffen dann vorzugsweise in Abhängigkeit von dem erfaßten pH-Wert.

Die Steuereinrichtung S ist vorzugsweise derart ausgeführt, daß die Zuführung von Kohlendioxid bzw. anderen Kohlenwasserstoffen so gesteuert wird, daß der pH-Wert des Mediums 3 zyklisch variiert und/oder im wesentlichen zwischen 6,5 und 8,5, vorzugsweise etwa zwischen 7 und 8, beträgt bzw. gehalten wird.

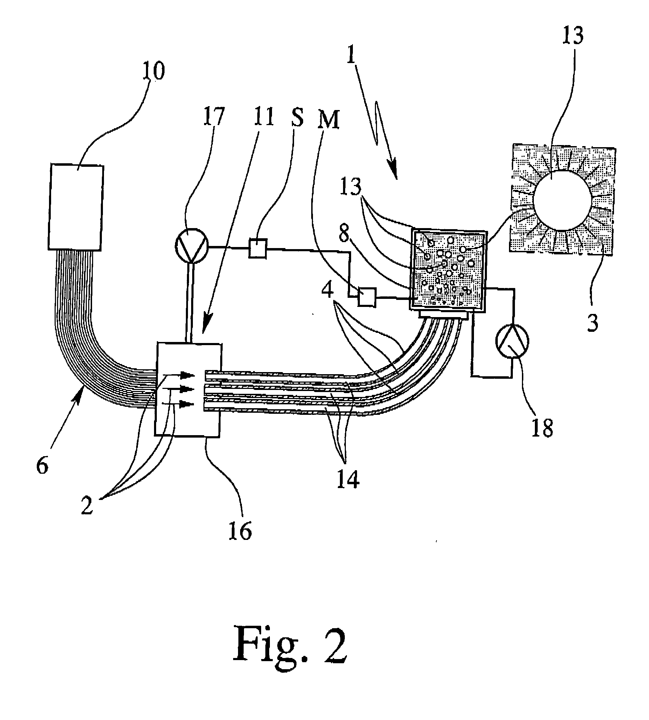

Nachfolgend wird eine zweite Ausftffimngsforrn der vorschlagsgemäßen Anordnung 1 und des vorschlagsgemäßen Verfahren zur Erzeugung von Biomasse anhand des Schemas gemäß Fig. 2 näher erläutert. Insbesondere werden nur wesentliche Unterschiede gegenüber der ersten Ausfiihrungsfonm beschrieben, so daß die bisherigen Ausführungen und Erläuterungen insbesondere entsprechend oder ergänzend gelten.

Bei der ersten Ausführungsform verzweigen sich die Fasern 4 bzw. Bündel 5, 6 insbesondere baumartig, um das Licht 2 in verschiedenen Raumbereichen innerhalb des Mediums 3 - also dreidimensional - in dem Medium 3 zu verteilen. Bei der zweiten Ausführungsform ist alternativ oder ergänzend vorgesehen, daß das Licht 2 über Gasblasen 13 eingeleitet und insbesondere dreidimensional in dem Medium 3 verteilt wird. Die Einleitung bzw. Erzeugung der Gasblasen 13 erfolgt vorzugsweise über hohle Fasern 4, kann aber auch auf sonstige Weise erfolgen.

Bei der zweiten Ausführungsform sind die Fasern 4 zur Zuleitung des Lichts 2 vorzugsweise zumindest abschnittsweise und/oder teilweise hohl ausgebildet, um nicht nur das Licht 2, sondern auch das vorzugsweise kohlendioxidhaltige Gas gleichzeitig zuführen zu können. Insbesondere erfolgt eine Lichtleitung im Mantel der hohlen Fasern 4, die in Fig. 2 nur schematisch, nicht maßstabsgerecht angedeutet sind.

Beim Einleiten des Gases in das Medium 3 bilden sich die Gasblasen 13. Die Gasblasen 13 weisen vorzugsweise anfänglich eine Größe von im wesentlichen 1 bis 10 µm, insbesondere 2 bis 5 µm, auf, Bei dieser Größe bilden die Gasblasen 13 besonders geeignete Resonanzkörper für das aufzunehmende Licht.

Die hohlen Fasern 4 geben gleichzeitig das Licht an ihren Enden in den Bereichen ab, in denen die Gasblasen 13 gebildet werden. So können die Gasblasen 13 quasi mit Licht 2 "gefüllt" werden. Es wird eine Art Nebel oder Molke leuchtender Gasblasen 13 erzeugt.

Die leuchtenden Gasblasen 13 - beispielhaft ist eine leuchtende Gasblase 13 in einer Ausschnittsvergrößerung in Fig. 2 dargestellt - wandern durch das Medium 3 nach oben. Hierbei werden die Gasblasen 13 aufgrund des abnehmenden Flüssigkeitsdrucks tendenziell größer. Andererseits kann sich das Gas im Medium 3 lösen bzw. von den Algen oder dergleichen aufgenommen werden, so daß die Gasblasen 13 dementsprechend tendenziell kleiner werden. Diese beiden Effekte können sich gegebenenfalls etwa kompensieren, so daß die Größe der Gasblasen 13 zumindest weitgehend gleichbleibt.

Um die gewünschte Gasblasengröße zu erreichen bzw. deren Bildung zumindest zu unterstützen, erfolgt die Einleitung des Gases vorzugsweise in einer Tiefe von etwa 3 bis 50 m, vorzugsweise 5 bis 10 m, in das Medium 3.

Die Einleitung des Gases erfolgt wiederum wie bei der ersten Ausführungsform vorzugsweise bodenseitig bzw. von unten.

Zur Formung der Gasblasen 13 bzw. Manipulation der Größen der Gasblasen 13 kann bedarfsweise ein Sieb 12 oder dergleichen eingesetzt werden.

Alternativ oder zusätzlich kann die gewünschte Gasblasengröße sehr einfach durch entsprechende Wahl des Innendurchmessers der hohlen Fasern 4 beeinflußt werden.

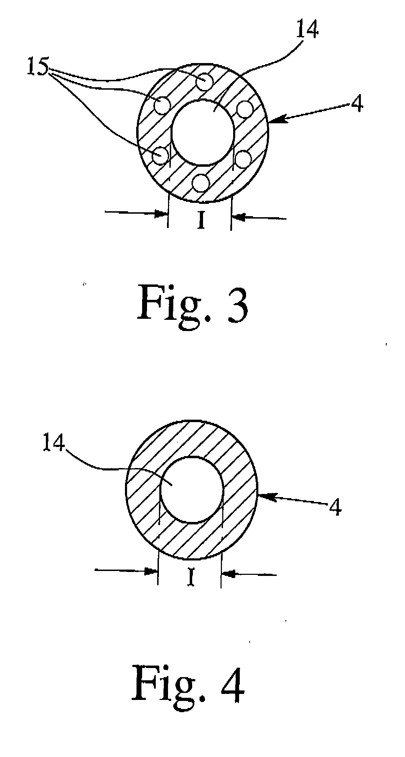

Fig. 3 und 4 zeigen beispielhaft zwei mögliche, unterschiedliche Querschnitte der hohlen Fasern 4, jeweils in schematischer, nicht maßstabsgerechter Darstellung.

Bei der Ausführungsvariante gemäß Fig. 3 handelt es sich um eine sogenannte Band-Gap-Faser. Die Gasführung erfolgt vorzugsweise ausschließlich über den Innen- bzw. Zentralkanal 14, bedarfsweise jedoch auch über die weiteren peripheren Hohlkanäle 15 dieser Faser 4. In diesem Fall erfolgt die Lichtleitung dann nur im Mantel der Faser 4.

Alternativ oder zusätzlich kann die Lichtleitung über die peripheren Hohlkanäle 15 erfolgen.

Fig. 4 zeigt eine "herkömmliche" Hohlfaser 4. Die Gaszuführung erfolgt hier über den Zentralkanal 14. Die Lichtleitung erfolgt im Mantel der Faser 4,

Um die gewünschte Gasblasengröße zu erreichen, beträgt der Innendurchmesser I der hohlen Fasern 4 bzw. Zentralkanäle 14 vorzugsweise höchsten 10 µm, insbesondere im wesentlichen 5 bis 9 µm.

Die Anordnung 1 bzw. Einrichtung 11 weist beim Darstellungsbeispiel vorzugsweise eine Zufiihreinrichtung 16 insbesondere mit einem unter Gasdruck stehendem Gehäuse auf, um das Gas in die hohlen Fasern 4 einzuleiten und das Licht 2 in die Fasern 4 einzukoppeln.

Das Gas wird beispielsweise über eine Pumpe 17 oder dergleichen zugeführt und tritt insbesondere stirnseitig in die Kanäle 14 bzw. 15 ein.

Das Licht 2 wird beispielsweise über lichtleitende Fasern 4 bzw. ein Faserbündel 6 oder dergleichen - insbesondere wiederum von einer Lichtsammeleinrichtung, wie dem in Fig. 2 nur schematisch angedeuteten Kollektorspiegel 10 o. dgl. - zugeführt bzw. zugeleitet. Fig. 2 zeigt die Ankopplung nur sehr schematisch. Insbesondere wird das Licht stirnseitig in die Anfänge der hohlen Fasern 4 eingeleitet.

Die Anordnung 1 weist wiederum vorzugsweise eine Meßeinrichtung M auf, um den pH-Wert des Mediums 3 im Tank 8 zu überwachen oder zu messen.

Die Anordnung 1 weist vorzugsweise wiederum eine Steuereinrichtung S auf, um den pH-Wert des Mediums 3 in der bereits eingangs und bei der ersten Ausführungsform erläuterten Art und Weise zu steuern bzw. zu regeln, insbesondere wiederum durch entsprechende Variationen oder Unterbrechung der Zuführung von Kohlendioxid bzw. kohlendioxidhaltigem Gas. Insbesondere steuert die Steuereinrichtung S hierzu die Pumpe 17, ein nicht dargestelltes Ventil o. dgl. bzw, die Versorgungseinrichtung 11, um die Zuführung von Kohlendioxid bzw. sonstigen Kohlenwasserstoffen als Steuergröße zur pH-Weit-Steuerung oder der bevorzugten pH-Wert-Regelung einzusetzen.

Dem Behälter oder Tank 8 bzw. dem Medium 3 ist vorzugsweise eine Umwälzeinrichtung, wie eine Umwälzpumpe 18, ein Rührwerk oder dergleichen, zugeordnet. So kann eine ausreichende und insbesondere das Wachstum fördernde Bewegung des Mediums 3 bzw. der Algensuspension sichergestellt werden.

Die Versorgungseinrichtung 11 dient besonders bevorzugt der Einleitung von vorzugsweise Kohlendioxid enthaltendem Gas, insbesondere Kohlendioxid, Rauchgas, Klärgas, Faulgas oder Luft, in das Medium 3. Jedoch können alternativ oder zusätzlich Kohlenwasserstoffe bzw. Kohlendioxid dem Medium 3 auch in flüssiger Form bzw. gelöster Form, beispielsweise durch Hinzugeben in den Umwälzkreis durch die Umwälzpumpe 18 o. dgl. zugegeben werden.

Die vorschlagsgemäße Anordnung 1 wird vorzugsweise derart betrieben, daß die Biomasseerzeugung besonders schnell erfolgt. Versuche haben gezeigt, daß dies durch die vorgeschlagene pH-Wert-Stcuerung bzw. -Regelung- insbesondere bei Algen als Spezies des Mediums 3 - erreicht wird.

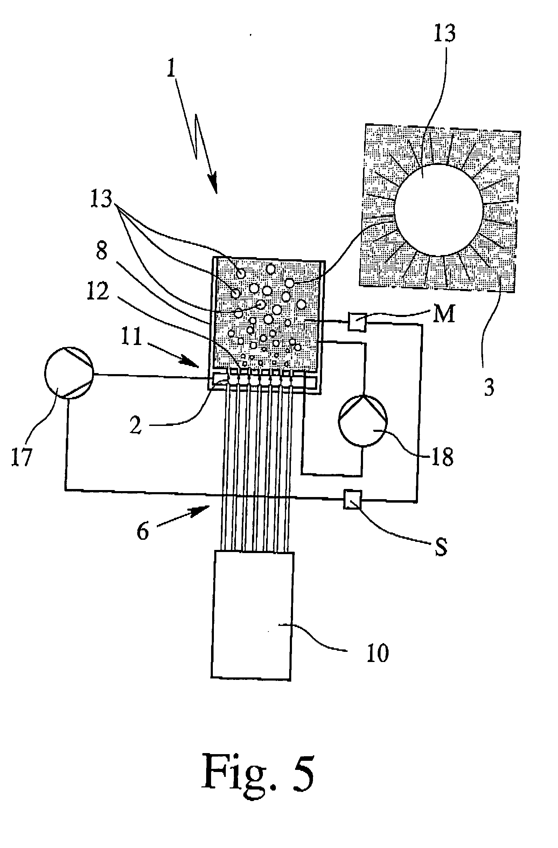

Fig. 5 zeigt in einer schematischen Darstellung eine dritte Ausführungsform der vorschlagsgemäßen Anordnung 1 bzw. Einrichtung 11. Hier erfolgt die Erzeugung und Einleitung der Gasblasen 13 vorzugsweise über ein Gitter, ein Sieb 12 einen Lochboden oder dgl. Insbesondere kann es sich auch um einen Zwischenboden handeln. So können die bei der zweiten Ausüffirungsform aufgrund der hohlen Fasern 4 auftretenden Druckverluste bei der Zuführung des Gases vermieden oder zumindest minimiert werden.

Bei der zweiten und dritten Ausführungsform erfolgt insbesondere die Lichteinkopplung in die Gasblasen 13 dadurch, daß das Gas durch kleine Löcher oder Kanäle in einem optisch transparenten bzw. lichtleitenden Material geleitet wird und beim Austritt die Gasblasen 13 bildet und Licht 2 aufnimmt.

Besonders bevorzugt ist das Sieb 12 bzw. der Lochboden aus einer Platte, und/oder transparentem Material vorzugsweise Kunststoff, insbesondere mit einer Dicke von etwa 3 bis 10 mm, hergestellt. Die Platte weist vorzugsweise eine Vielzahl kleiner Löcher mit einem Durchmesser von höchstens 50 µm auf. Das Gas tritt durch diese Löcher hindurch in das Medium 3 ein. Die Beleuchtung erfolgt vorzugsweise durch das transparente Plattenmaterial und/oder gasseitig. Über die Länge der Löcher bzw. Bohrungen - also über die Dicke der Platte - werden quasi "hohle Lichtwellenleiter" mit dem strömenden Gas als Innenraum und der Bodenplatte als optischem Mantel gebildet. So können die entstehenden Gasblasen 13 optisch an das Lichtfeld bzw. Licht 2 ankoppeln bzw. umgekehrt kann so das Licht 2 von den Gasblasen 13 aufgenommen werden.

Bei der dritten Ausführungsform gelten ansonsten die Erläuterungen und Anmerkungen zur zweiten Ausführungsform insbesondere entsprechend oder ergänzend. Insbesondere kann wieder ein Kollektorspicgel 10 oder jede sonstige geeignete Beleuchtungseinrichtung zur Zuleitung von Licht - optional wieder über Faserbündel 6 oder dgl. - eingesetzt werden.

Alternativ oder zusätzlich kann das Licht 2 jedoch auch auf sonstige Art und Weise, insbesondere ggf. auch seitlich, in die optisch lichtdurchlässige bzw. transparente Platte mit den Löchern, über die das Gas in das Medium 3 zur Bildung der Gasblasen 13 eingeleitet wird, angekoppelt werden.

Bei der dritten Ausführungsform können bedarfsweise auch zusätzlich an einigen Stellen des Bodens Rohre oder sonstige Vorsprünge, Verlängerungen oder dgl., beispielsweise mit einer Länge von etwa 1 bis 2 m, angebracht werden oder nach oben abragen bzw. vorspringen bzw. angeschlossen sein, in denen Lichtleitkabel geführt werden. Die Stirnfläche dieser Rohre kann dann beispielsweise mit einer Lochkonstruktion bzw. einem Aufbau, wie die Bodenplatte ausgebildet werden. So kann die Begasung und Belichtung des Reaktorvolumens bzw. des Mediums 3 vergleichmäßigt werden. Alternativ oder zusätzlich können auch mehrere Zwischenböden, die entsprechend der zweiten oder dritten Ausführungsform ausgebildet sind und beispielsweise in unterschiedlichen Höhen bzw. Tiefen angeordnet oder versetzt werden, eingesetzt werden.

Gemäß einer weiteren Ausführungsvariante werden wesentlich kleinere Gasblasen 13 gebildet. Der anfängliche Durchmesser der Gasblasen 13 beträgt beispielsweise 0,1 bis 10 µm, vorzugsweise weniger als 1 µm, insbesondere im wesentlichen 0,2 bis 0,6 µm.

Derartig kleine Gasblasen 13 können insbesondere durch Einleitung in einer entsprechenden Tiefe, beispielsweise etwa 50 m oder mehr, in das Medium 3 erfolgen. Alternativ oder zusätzlich kann das Medium 3 auch unter Druck gesetzt werden,

Besonders bevorzugt erfolgt die Einleitung des Gases bei einem Druck des Mediums 3 von etwa 50 bis 800 kPa. Besonders bevorzugt beträgt der Druck des Mediums 3, bei dem das Gas eingeleitet wird, mindestens etwa 500 kPa und mehr, um sehr kleine Gasblasen 13 zu bilden.

Aufgrund der geringen Größe der Gasblasen 13 ist es möglich, diese durch direkte Belichtung mit Licht, insbesondere Sonnenlicht, zu Resonanzabsoxption anzuregen, so daß das Licht beim Aufsteigen der Gasblasen 13 ins Reaktorinnere bzw. nach oben durch das Medium 3 transportiert wird. Die Belichtung der Gasblasen 13 kann insbesondere wieder über Lichtleitfasern 4 und/oder auf sonstige Art und Weise erfolgen.

Es ist anzumerken, daß die Gasblasen 13 Resonatoren für das eingestrahlte Licht 2 bilden können. Die Resonanzfrequenzen hängen von dem Durchmesser der Gasblasen 13 ab. Um nicht nur monochromatisches Licht aufnehmen zu können, werden vorzugsweise Gasblasen 13 mit unterschiedlichen Durchmessern, insbesondere mit einem das zugeführte Lichtspektrum abdeckenden Durchmesserspektrum, erzeugt.

Bei den beschriebenen Ausführungsformen kann die Photosynthese mittels einer technischen Einrichtung nachgeahmt werden, nämlich aus Kohlendioxid und Wasser unter Einwirkung von Licht Biomasse bzw. CH₂O-enthaltene Verbindungen und Sauerstoff zu erzeugen. Dementsprechend kann die vorschlagsgemäße Anordnung 1 auch als Bioreaktor, insbesondere als faseroptischer Photo-Bioreaktor arbeiten bzw. bezeichnet werden.

Besonders bevorzugt wird Sonnenlicht verwendet, das insbesondere mittels Konzentratoren, Kollektorspiegeln 10 oder dergleichen gesammelt und dem Medium 3 zugeleitet und insbesondere in dieses eingeleitet wird, wie bereits anhand der beiden Ausführungsformen erläutert. Alternativ oder zusätzlich kann das Sonnenlicht auch durch ein Netz von Fluoreszenzfasem gesammelt werden.

Das konzentrierte Sonnenlicht oder sonstiges Licht wird vorzugsweise in optische Lichtleitfasern - bei der vorliegenden Erfindung kurz als 4 bezeichnet - eingespeist und insbesondere in Form eines Bündels 5/6 dem Bioreaktor, hier also dem Behälter bzw. Tank 8 mit dem Medium 3, zugeleitet.

Besonders bevorzugt kann die Algensuspension im gesamten Reaktorvolumen, also im gesamten Behälter 8, mittels der Fasertechnik beleuchtet werden. Insbesondere erfolgt dies dadurch, daß die Fasern 4 in unterschiedlichen Raumbereichen im Behälter 8 enden bzw. sich verzweigen, wie anhand der ersten Ausführungsform erläutert. Alternativ oder zusätzlich kann die räumliche Verteilung des Lichts 2 in dem Medium 3 auch mittels Gasblasen 13, insbesondere durch einen Nebel aus leuchtenden Bläschen erfolgen, wie anhand der zweiten Ausführungsform erläutert.

Damit wird eine dreidimensionale Beleuchtung erreicht. Bei herkömmlicher Beleuchtung von einer Seite bzw. zumindest im wesentlichen nur aus einer Ebene wird wegen der hohen Schwächungskonstante der Algensuspension hingegen nur eine Zone von wenigen mm Eindringtiefe erreicht.

Wie bereits erläutert, kommt den Lichtleitfasern bei der vorliegenden Erfindung eine zweifache Bedeutung zu, Die einzelnen Effekte können auch unabhängig voneinander eingesetzt bzw. ausgenutzt werden.

Erstens dienen die Fasern 2 einem effektiven Lichttransport über große Längen. Bei großen anfallenden Kohlendioacidmengen, wie bei einem Kohlekraftwerk, wird sehr viel Licht zur Kohlendioxid-Bindung benötigt. Das erfordert entsprechend große Flächen zum Sammeln des Sonnenlichts mit entsprechend langen Transportwegen von über einigen 100 m.

Zweitens dienen die Lichtleitfasern 4 der Ausleuchtung eines dreidimensionalen Volumens im Behälter 4. Dies erfolgt durch die vorzugsweise baumartige Verzweigung und/oder mittels der Gasblasen 13.

Diesen Prinzipien machen die Anordnung 1 bzw. den Bioreaktor skalierbar.

Die beschriebene Erfindung soll helfen, das Kohlendioxidproblem zu lösen. Insbesondere sind die vorschlagsgemäße Anordnung 1 und das vorschlagsgemäße Verfahren zum Abbau von Kohlendioxid aus dem Rauchgas fossiler Kraftwerke geeignet.

Zum Abbau von Kohlendioxid aus Rauchgas kann das Rauchgas gegebenenfalls einfach von unten in den Behälter 8 eingeblasen werden, wie bei den beiden Ausfuhrungsformen angedeutet.

Ein weiterer Vorteil der vorliegenden Erfindung liegt darin, daß nicht nur Kohlendioxid abgebaut bzw. gebunden werden kann, sondern daß die erzeugte Biomasse auch als Rohmaterial für Brennstoff, Bioethanol, Biodiesel oder dergleichen verwendet werden kann.

Die starke Streuung des Mediums 3 unterstützt eine zumindest weitgehend gleichmäßige oder vollständige Ausleuchtung des Mediums 3 bzw. des Behälters 8.

Es ist anzumerken, daß von dem Sonnenlicht bedarfsweise der IR-Anteil abgetrennt, und beispielsweise für sonstige Zwecke, insbesondere zur Trocknung der erzeugten Biomasse, wie der Algen, oder dergleichen, eingesetzt werden kann.

Einzelne Merkmale und Aspekte der erläuterten Ausfuhrungsfbrmen und -varianten können auch beliebig miteinander kombiniert und/oder bei sonstigen Anordnungen oder Verfahren eingesetzt werden.

## Patentansprüche

1. Anordnung (1) zur Biomasseerzeugung, mit einem Medium (3) mit mindestens einer lebenden Spezies, insbesondere Algen, mit einer Beleuchtungseinrichtung zur Zuführung von Licht (2) und/oder zur Verteilung von Licht (2) in dem Medium (3), und mit einer Versorgungseinrichtung (11) zur Zuführung von Kohlendioxid und/oder anderen Kohlenwasserstoffen,
**dadurch gekennzeichnet,**
**daß** die Anordnung (1) eine Steuereinrichtung (S) zur Steuerung oder Regelung des pH-Werts des Mediums (3) und/oder zur Steuerung der Versorgungseinrichtung (11) und/oder der Zuführung von Kohlendioxid und/oder anderen Kohlenwasserstoffen aufweist.

2. Anordnung nach Anspruch 1, **dadurch gekennzeichnet, daß** die Anordnung (1) eine Meßeinrichtung (M) zur Erfassung des pH-Werts des Mediums (3) aufweist.

3. Anordnung nach Anspruch 2, **dadurch gekennzeichnet, daß** die Steuereinrichtung (S) zur Steuerung der Zuführung von Kohlendioxid und/oder anderen Kohlenwasserstoffen in Abhängigkeit von dem erfassten pH-Wert ausgebildet ist,

4. Anordnung nach einem der voranstehenden Ansprüche, **dadurch gekennzeichnet**, die Steuereinrichtung (S) derart die Zuführung von Kohlendioxid und/oder anderen Kohlenwasserstoffen steuert, daß der pH-Wert des Mediums (3) zyklisch variiert und/oder im wesentlichen zwischen 6,5 und 8,5, vorzugsweise zwischen 7 und 8, beträgt.

5. Anordnung nach einem der voranstehenden Ansprüche, **dadurch gekennzeichnet, daß** die Steuereinrichtung (S) zur Regelung des pH-Werts des Mediums (3) durch Variation und/oder Unterbrechung der Zuführung von Kohlendioxid und/oder anderen Kohlenwasserstoffen ausgebildet ist.

6. Verfahren zur Biomasseerzeugung, wobei einem Medium (3) mit mindestens einer lebenden Spezies, insbesondere Algen Kohlendioxid und/oder anderen Kohlenwasserstoffe zugeführt werden,
**dadurch gekennzeichnet,**
**daß** der pH-Wert des Mediums (3) zwischen 6,5 und 8,5 gehalten, zyklisch variiert und/oder durch Variieren und/oder Unterbrechen der Zuführung von Kohlendioxid und/oder anderen Kohlenwasserstoffen gesteuert oder geregelt wird.

7. Verfahren nach Anspruch 6, **dadurch gekennzeichnet, daß** dem Medium (3) Kohlendioxid gasförmig - insbesondere in Form kleiner Gasblasen (13) - zugeführt wird.

8. Verfahren nach Anspruch 6 oder 7, **dadurch gekennzeichnet, daß** das Medium (3) beleuchtet wird.
